# EUROPEAN PATENT APPLICATION

(11) **EP 4 487 917 A2**
(43) Date of publication of application: **08.01.2025**
(21) Application number: 24200474.5
(22) Date of filing: 16.07.2020
(51) Int. Cl.: A61P 31/04

(54) **STABILIZING THERAPEUTIC PROTEINS WITH PIPERAZIN- OR MORPHOLINE-CONTAINING ZWITTERIONIC BUFFERING SUBSTANCES**

(30) Priority: 29.07.2019 EP 19188904
(62) Divisional of application: 20740014.4
(71) Applicant: Ludwig-Maximilians-Universität München, 80539 München (DE); HYpharm GmbH, 82347 Bernried (DE)
(72) Inventor: WINTER, Gerhard, 82377 Penzberg (DE); EISELE, Simon, 80992 Munich (DE); MOLINARO, Sonja, 82362 Weilheim (DE); MUTTER, Wolfgang, 82347 Bernried (DE)
(74) Representative: Grünecker Patent- und Rechtsanwälte PartG mbB

(57) **Abstract**

The present invention relates to compositions comprising a therapeutic protein with enhanced stability, which comprise a piperazine- or morpholine-containing zwitterionic buffering substance, such as HEPES, in particular when used out of the common pH range. The compositions of the invention are particularly useful for topical administration. The present invention further relates to the use of said compositions for treating bacterial infections, e.g. bacterial infections caused by Staphylococcus aureus such as methicillin-resistant Staphylococcus aureus (MRSA). The present invention further relates to the use of said compositions for preventing or eliminating nasal bacterial colonization or bacterial colonization of the skin.

## Description

### TECHNICAL FIELD

The present invention relates to compositions with enhanced stability, which are suitable for topical administration of a therapeutic protein, such as a bacteriophage lysin, wherein the compositions comprise a piperazine- or morpholine-containing zwitterionic buffering substance for stabilization, and optionally one or more pharmaceutically acceptable carriers or excipients. The present invention further relates to the use of said compositions for treating bacterial infections, e.g. bacterial infections caused by *Staphylococcus aureus* such as methicillin-resistant *Staphylococcus aureus* (MRSA). The present invention further relates to use of said compositions for preventing or eliminating nasal bacterial colonization or skin colonization. The present invention further relates to methods for preparing the stabilized compositions.

### BACKGROUND OF THE INVENTION

In general, therapeutic proteins are relatively unstable during storage, and this can result in decomposition of the active agent and loss of the desired therapeutic effect. Moreover, there is a risk that parenteral or topical administration of the degraded active agent may cause immunogenic activity (Jiskoot *et al.,* 2012). A stable formulation of a therapeutic protein is, thus, the basis for the successful development of a medicament over the period up to regulatory approval.

With respect to the protein formulation, a distinction must be made between physical and chemical instability. For instance, physical instability is expressed in denaturation, which also means the loss of the tertiary structure of the proteins. Moreover, the proteins can aggregate, which means that native and modified (conformational) protein monomers may form an association. Chemical modifications may occur alone or in combination with physical changes. Examples include deamination of Asn and Gln, hydrolysis of Asp and Trp, and oxidation. Oxidation is the major cause of instability, and may occur in any protein that contains oxidizable amino acids such as His, Met, Cys, Tyr and Trp. Caused by this oxidation, cysteine disulphide bridges and sulfonyl species may be formed (Shire, 2015).

In order to avoid the instability of therapeutic proteins (e.g., oxidation, the most frequently occurring cause for this instability), various approaches have been developed. Firstly, the influence of external factors, such as UV radiation or oxygen content, may be reduced by using specific packaging agents or storage conditions. Furthermore, additives (e.g., antioxidants) may be added, which themselves will be oxidized during storage and thus prevent oxidation of the therapeutic proteins (Hada *et al.,* 2016).

Various additives may be used for improving physical stability, especially conformational and colloidal stability. These include, among others, buffering substances, which are usually used to control pH values. Surfactants are also frequently used to prevent aggregation and denaturation, as well as to prevent any formation of particles.

The selection of an appropriate buffering system depends on various factors; e.g., pH value of the formulation and the addition of excipients. The control of the pH values by means of an appropriate buffering system may also have an influence on the solubility and stability of the protein. Common buffering systems that are frequently used in protein formulations contain phosphate, acetate, citrate, succinate, histidine, glycine, arginine, triethanolamine and maleate. Among these buffering systems, citrate, succinate and histidine are particularly suitable for the application, with a pH value of 6.0. However, due to their incompatibility with Ca²⁺-, Zn²⁺-, or other divalent cations, buffering systems including phosphate are often not suitable to be applied to various formulations. Therefore, additives like CaCl₂, which may be used in connection with the compositions disclosed in the present application, lead to a more complex formulation that is inapplicable with most of the common buffering systems (Banga, 2006).

Sugar and sugar alcohols are also frequently used as stabilizers.(Jorgensen, Hostrup, Moeller, & Grohganz, 2009).

In formulations containing therapeutic proteins, buffering substances are widely used; however, they are used exclusively in aqueous preparations, such as the recently described self-buffering systems. Buffering substances to be applied in therapeutic formulations are described in numerous publications and textbooks. In this context, it is nevertheless obvious that only very few buffer substances are actually applicable in protein drugs, in particular phosphate, citrate, acetate, succinate, TRIS, HIS, and glycine. Mixtures of these buffering substances are also commonly used in the pharmaceutical area, in particular citrate-phosphate buffer; however, other buffering substances are seldom used in this area.

Moreover, the buffering substances are mainly used within an optimal range of pH, which is within ± one pH unit of their pKa value. It has long been known that physical stability is influenced by the selection of the specific buffering substance. In particular, the classification of buffer cations and anions according to the so-called "Hoffmeister" series, and their corresponding effect on solubility and conformational stability, has long been recognized.

In contrast to the above-mentioned frequently-used buffer substances, piperazine- or morpholine-containing zwitterionic buffering substances, such as 2-(4-(2-hydroxyethyl)-1-piperazinyl)-ethane sulfonic acid (HEPES), are essentially not used as buffer substances for pharmaceutical products - or even for formulations of therapeutic proteins. The list of pharmaceutical preparations for antibodies recently compiled by Dr. Abina Crean (Senior Lecturer in Pharmaceutics) does not recite piperazine- or morpholine-containing zwitterionic buffering substance, e.g., HEPES.

The few cases in which HEPES or other piperazine- or morpholine-containing zwitterionic buffering substances are cited mention these substances in connection with pharmaceutical formulations. However, following established practice with respect to buffers, they are used in the range of their optimal buffer capacity; i.e. close to the pKa value of their corresponding free acid.

For instance, US 587.699.2 A discloses the use of 50 mM HEPES (piperazine-containing zwitterionic buffering substance) at pH 7.5. As an alternative, the authors suggest the use of TRIS and phosphate-using substances, or a combination thereof.

US 2015/0071925 A1 describes the use of a piperazine- or morpholine-containing zwitterionic buffering substance (i.e., HEPES) as viscosity-reducing substance in highly concentrated Avastin-containing pharmaceutical formulations, which is in clear contrast to the compositions of the present invention, wherein Hy 133 is formulated in fluid or semisolid compositions with increased viscosity.

EP 0 988 861 A1 describes the use of a piperazine- or morpholine-containing zwitterionic buffering substance (i.e., HEPES, alternatively TES and TRICINE), which has a stabilizing effect on G-CSF in concentrations of 1 M or higher within a pH range of 4-7.5, preferably pH 7.5. The concentration where HEPES or alternatively TES and TRICINE have a stabilizing effect (1M or higher) lies clearly far above the typical concentrations at which buffers are used when formulating protein drugs.

There is an ongoing need for therapeutic compositions comprising therapeutic proteins that decompose easily in common buffering systems. In particular, there is a need for therapeutic compositions comprising recombinantly produced chimeric bacteriophage lysins. Further, there is a need for therapeutic compositions comprising buffering systems providing an improved storage stability by reducing or avoiding degradation of the active agent. A major problem is that common buffering systems for therapeutic compositions, when used in the range of their optimal buffer capacity, have a detrimental effect on stability due to degradation of the therapeutic protein. In particular, there is a need for buffering systems suitable for topical administration.

Accordingly, there is a commercial need for new therapeutic compositions comprising therapeutic proteins - especially those that contain bacteriophage lysins, which are suitable for topical administration and have an increased storage stability.

### SUMMARY OF THE INVENTION

The inventors surprisingly found that piperazine- or morpholine-containing zwitterionic buffering substances, such as 2-(4-(2-hydroxyethyl)-1-piperazinyl)-ethane sulfonic acid (HEPES), are highly useful as stabilizers for therapeutic proteins such as bacteriophage lysins - in particular when used out of the common pH range. The inventors found that degradation of therapeutic proteins could be avoided by the use of these buffering substances, which were normally not used in combination with therapeutic proteins. Therefore, the core of the invention is the application of piperazine- or morpholine-containing zwitterionic buffering substances to stabilize liquid protein compositions.

Moreover, the inventors describe composition, e.g. gel-like or thickened compositions, for topical administration of therapeutic proteins such as bacteriophage lysins, in particular compositions prepared for cutaneous or nasal administration.

The present invention provides the following items:
[1] Composition for topical administration of a bacteriophage lysin, preferably a pharmaceutical composition comprising:
   (I) a bacteriophage lysin as an active ingredient,
   (II) a piperazine- or morpholine-containing zwitterionic buffering substance, and
   (III) optionally a pharmaceutically acceptable carrier or excipient.
[2] The composition of item 1, wherein the piperazine- or morpholine-containing zwitterionic buffering substance is selected from the group consisting of 2-(4-(2-hydroxyethyl)-1-piperazinyl)-ethane sulfonic acid (HEPES), piperazine-1,4-bis(2-hydroxy-3-propane sulfonic acid) dehydrate (POPSO), 2-(N-morpholino)-ethane sulfonic acid (MES) and 3-(N-morpholino)-propane sulfonic acid (MOPS), preferably wherein the composition comprises HEPES as the sole buffer.
[3] The composition of item 1 or 2, wherein the bacteriophage lysin is a polypeptide comprising a first portion and a second portion joined by a linker, wherein said first portion comprises an amino acid sequence of a bacteriocin cell-binding domain (CBD) and said second portion comprises an amino acid sequence of an enzymatic active domain (EAD).
[4] The composition of any one of items 1 to 3, wherein the EAD is the lytic domain of a bacteriophage endolysin.
[5] The composition of item 4 , wherein the lytic domain has at least 80%, preferably 90%, amino acid sequence identity with the polypeptide of SEQ ID NO: 1, and/or, wherein the CBD has at least 80%, preferably 90%, amino acid sequence identity with the polypeptide of SEQ ID NO: 2.
[6] The composition of any one of items 1 to 5, wherein the bacteriophage lysin is a polypeptide having at least 80%, preferably 90%, amino acid sequence identity with the polypeptide of SEQ ID NO: 5.
[7] The composition of any one of items 1 to 6, wherein the bacteriophage endolysin is lysK.
[8] The composition of any one of items 1 to 7, wherein the CBD is a lysostaphin CBD.
[9] The composition of any one of items 1 to 8, wherein the composition is in form of a solution, a gel-like preparation, a semi-solid or solid preparation, a sprayable preparation or a lyophilizate.
[10] The composition of any one of items 2 to 9, wherein the composition is in form of a solution comprising HEPES but does not comprise additional buffers.
[11] The composition of one of items 1 to 9, wherein the composition is in form of a solution comprising HEPES and one or more other excipients, and wherein the pH value of the solution is in the range of pH 4-8, preferably in the range of pH 5-7, more preferably in the range of 5.5-6.5.
[12] The composition of any of items 1 to 11, wherein the composition comprises antioxidants, preferably Methionin, and one or more other excipients, preferably selected from the group consisting of CaCl₂, NaCl, Arginin-HCl, and Poloxamer 188.
[13] The composition according to item 11 or 12, wherein the composition comprises HEPES, Methionin, CaCl₂, NaCl, and Arginin-HCl, and the pH of the composition is pH=6.
[14] The composition of any one of items 2 to 13, wherein the HEPES concentration is in the range of 0.01-1000 mM, preferably in the range of 1-400 mM, more preferably in the range of 10-100 mM, even more preferably in the range of 20-40 mM.
[15] The composition of any one of items 1 to 13, wherein the composition further comprises HPMC, preferably HPMC K4M, K15M or K100M
[16] The composition according to item 15, comprising 0.1-4% per weight HPMC, preferably 0.5-2 % per weight HPMC K4M.
[17] The composition of any one of items 1 to 16, wherein the bacteriophage lysin concentration is in the range of 0.01 mg/ml to 100 mg/ml, preferably in the range of 0.1 mg/ml to 20 mg/ml, more preferably in the range of 0.2 mg/ml to 10 mg/ml, even more preferably in the range of 0.4 mg/ml to 6 mg/ml, and most preferred in the range of 0.5 mg/ml to 2 mg/ml.
[18] The composition of any one of items 1 to 17, wherein the composition is prepared for cutaneous or nasal administration.
[19] The composition according to any one of items 1 to 18 for use in treating a bacterial infection or preventing or eliminating nasal bacterial colonization or skin colonization.
[20] The composition for use according to item 19, wherein the bacterial infection or colonialization comprises Staphylococcus, preferably Staphylococcus aureus.
[21] The composition according to any one of items 1 to 20 for use in treating MRSA or for ameliorating wounds.
[22] A method for preparing a composition for topical administration of a bacteriophage lysin according to any of items 1 to 18 comprising the step of mixing a bacteriophage lysin solution with a zwitterionic buffering substance and optionally adding a pharmaceutically acceptable carrier or excipient.

### BRIEF DESCRIPTION OF THE DRAWINGS

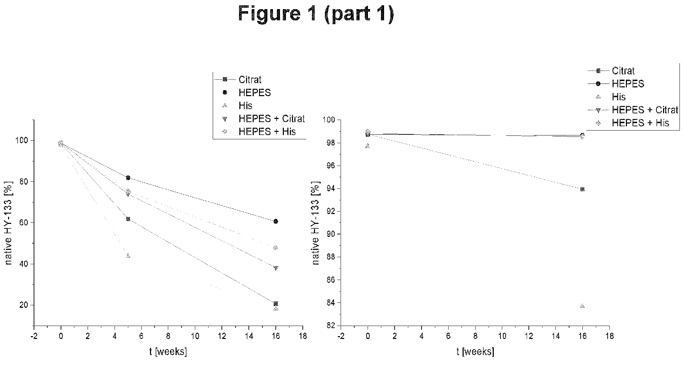

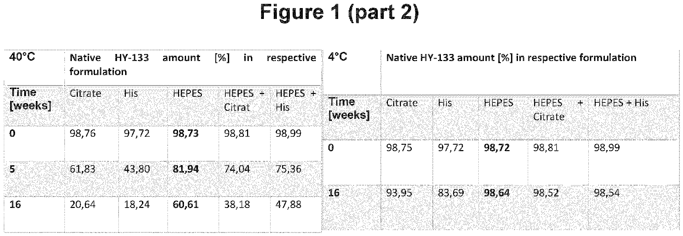

Figure 1: The graphical representation and the table on the left side of Figure 1 show the concentration of native HY-133 after 16 weeks' storage at 40°C. The graphical representation and the table on the right side of Figure 1 shows the concentration of native HY-133 after 16 weeks' storage at 4°C. (Example 2).

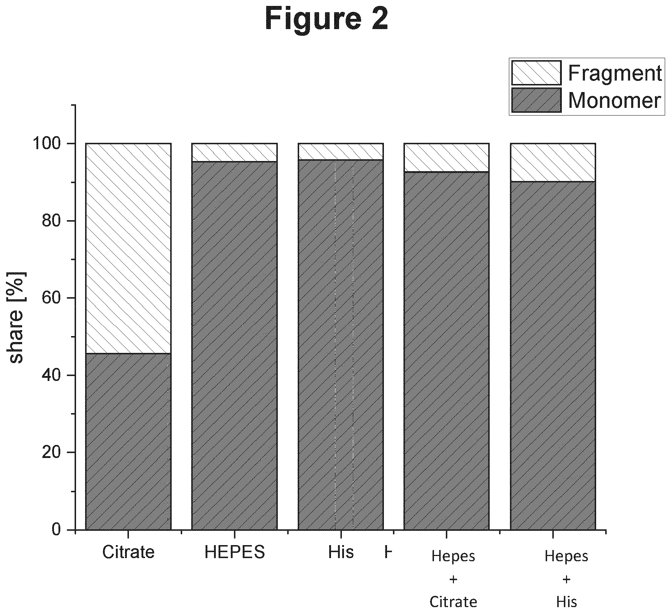

Figure 2 shows the degree of fragmentation (percentage) of various HY-133 preparations after five weeks' storage at 40°C (Example 3).

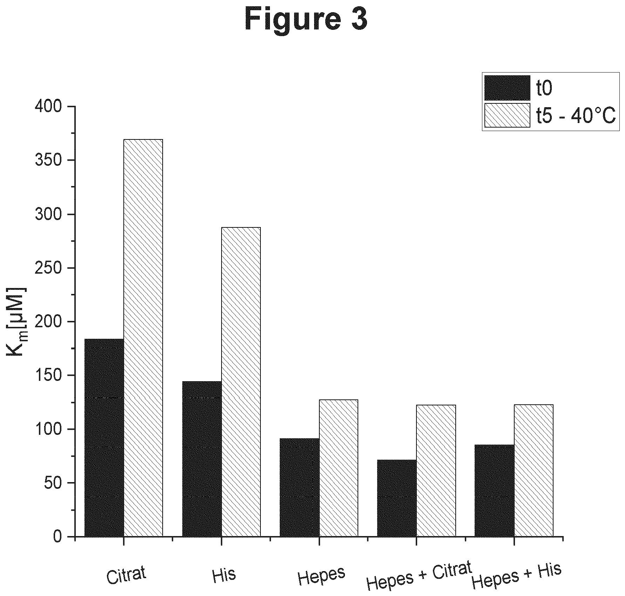

Figure 3 shows the substrate affinity (Km value) of various HY-133 preparations (i.e. in citrate, His, HEPES, citrate/HEPES and His/HEPES buffer) after five weeks' storage at 40°C (Example 4).
Figure 4 shows the native protein concentration of HY-133 (A) and the specific activity (B) in a gel-like composition compared to a liquid control composition after six months' storage at 4°C (Example 5).

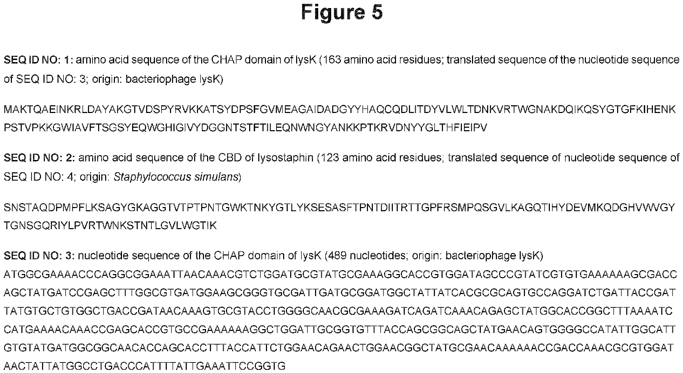

Figure 5 shows the amino acid sequence of the CHAP domain of lysK (SEQ ID NO:1); the amino acid sequence of the CBD of lysostaphin (SEQ ID NO:2); and the nucleotide sequence of the CHAP domain of lysK (SEQ ID NO:3).

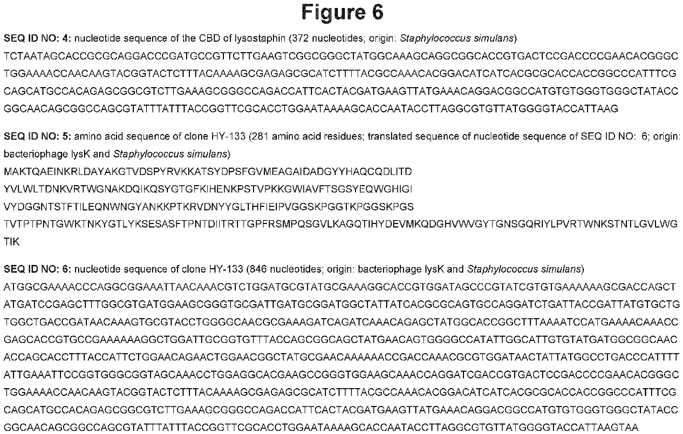

Figure 6 shows the nucleotide sequence of the CBD of lysostaphin (SEQ ID NO:4); the amino acid sequence of clone HY-133 (SEQ ID NO:5); and the nucleotide sequence of clone HY-133 (SEQ ID NO:6).

### DETAILED DESCRIPTION OF THE INVENTION

This invention allows the preparation of compositions, in particular fluid compositions, comprising therapeutic proteins, such as bacteriophage lysins with an improved stability. The inventors surprisingly demonstrated that fluid compositions comprising antimicrobial active substances, such as bacteriophage lysins, may be stabilized by means of piperazine- or morpholine-containing zwitterionic buffering substance, which are usually not used in combination with therapeutic proteins, since therapeutic proteins, such as bacteriophage lysins, tend to disintegrate in the presence of the buffer substances - especially under conditions of their optimal buffer capacity. The inventors surprisingly found that these buffers are particularly useful when they are used outside of their optimal pH range.

The inventors surprisingly found that piperazine- or morpholine-containing zwitterionic buffering substances, such as HEPES, are necessary to stabilize protein formulations, even though the pH value of protein formulations is typically outside the optimal buffer area of these buffers (namely about 6.0), whereas the optimal buffer area of HEPES is 6.8-8.2.

Moreover, the inventors surprisingly showed that the selection of piperazine- or morpholine-containing zwitterionic buffering substances such as HEPES (compared to buffers commonly used in this pH range, e.g. citrate and histidine) has a positive effect on the specific activity of the bacteriophage lysin. Example 4 demonstrates that the activity of various HY-133 preparations is improved.

In this context, the terms "stability" and "stabilized", as used herein, mean time-dependent chemical and physical integrity of the protein after storage under variable storage conditions. Moreover, as used herein, the term "stability" means the ability to substantially retain the biological activity of the active agent (herein also referred to as therapeutic protein or phage lysin) included in the composition during the time of storage or use. In the present invention, the piperazine- or morpholine-containing zwitterionic buffering substance serves as a stabilizing buffer for the active agent.

In one embodiment, the invention provides a composition for topical administration of a bacteriophage lysin, preferably a pharmaceutical composition comprising: (I) a bacteriophage lysin as an active ingredient, (II) a piperazine- or morpholine-containing zwitterionic buffering substance as a stabilizer and (III) optionally a pharmaceutically acceptable carrier or excipient.

### Buffering substances/buffers

In one embodiment of the invention, the buffering substance(s) included in the composition of the invention is/are one or more piperazine- or morpholine-containing zwitterionic buffering substance(s) and optionally a pharmaceutically acceptable carrier or excipient, preferably one piperazine- or morpholine-containing zwitterionic buffering substance and optionally a pharmaceutically acceptable carrier or excipient. Suitable carriers or excipients are known in the art (Kamerzell, Esfandiary, Joshi, Middaugh, & Volkin, 2011). Examples for particularly suitable carriers or excipients are described below.

As used herein, "buffering substances" or "buffers" according to the invention refer to buffers comprising piperazine or morpholine entities. More specifically, such buffers according to the invention have zwitterionic character. As used herein, "zwitterionic buffering substance", or "zwitterionic buffer", refers to buffering substances or buffers exhibiting a zwitterionic character (e.g., does not possess a net charge, lacks conductivity and electrophoretic mobility, does not bind ion-exchange resins, breaks protein-protein interactions) including, but not limited to, 2-(4-(2-hydroxyethyl)-1-piperazinyl)-ethane sulfonic acid (HEPES), 3-(N-morpholino)-propane sulfonic acid (MOPS), piperazine-1,4-bis(2-hydroxy-3-propane sulfonic acid) dehydrate (POPSO), 2-(N-morpholino)-ethane sulfonic acid (MES), 2-[[1,3-dihydroxy-2-(hydroxymethyl)propan-2-yl]amino]ethanesulfonic acid (TES) or 3-(Bis(2-hydroxyethyl)amino)-2-hydroxypropane-1-sulfonic acid (DIPSO). Further suitable buffers having zwitterionic character are known in the art (Zbacnik *et al.,* 2017).

As used herein, "buffer capacity" is defined as the moles of an acid or base necessary to change the pH of a solution by 1, divided by the pH change and the volume of buffer in liters; it is a unitless number. A buffer resists changes in pH due to the addition of an acid or base though consumption of the buffer. Thus, the term "buffer capacity (β)" as used herein means the quantity of strong acid or strong base (in the buffer solution) that gives rise to a change of one pH unit in 1 L of solution. Such a buffer capacity can be imparted by adding a pH buffering substance or the like to the composition. The optimal buffer capacity according to the invention is close to the pKa value of the corresponding free acid.

In the present invention, piperazine- or morpholine-containing zwitterionic buffering substances, such as 2-(4-(2-hydroxyethyl)-1-piperazinyl)-ethane sulfonic acid (HEPES), piperazine-1,4-bis(2-hydroxy-3-propane sulfonic acid) dehydrate (POPSO), 2-(N-morpholino)-ethane sulfonic acid (MES) and 3-(N-morpholino)-propane sulfonic acid (MOPS) are particularly preferred. In the present invention, the composition may comprise HEPES, but is substantially free of additional buffers, preferably the composition of the invention is free of buffers selected from the group consisting of Acetate-, Phosphate-, Histidine-, Tris-, Citrate-, Citrate-phosphate- and Histidine-acetate buffer. As used herein, the term "substantially free of additional buffers" means that the composition contains less than 10 percent per volume, preferably less than 5 percent per volume, more preferably less than 1 percent per volume additional buffers.

As used herein, HEPES is provided as a non-exclusive example for the purpose of describing embodiments of the invention, and should not be construed as limiting the invention.

### Active agents/therapeutic proteins

The composition of the invention comprises a bacteriophage lysin as an active agent. A "phage" or "bacteriophage", as used herein, relates to the well-known category of viruses that infect bacteria. Phages include DNA or RNA sequences encapsidated in a protein envelope or coat ("capsid").

A "bacteriophage lysin" according to the present invention is a polypeptide, specifically a chimeric polypeptide comprising a combination of a first portion and a second portion, wherein the first portion comprises an amino acid sequence of a bacteriocin cell binding domain (CBD) and the second portion comprises at least one enzymatic active domain (EAD), wherein the domains stem from a different source or different origin.

In the present invention, the term "EAD" represents the abbreviation for enzymatic active domain, and the term "CBD" represents the abbreviation for cell binding domain, more specifically cell wall binding domain. Thus, the term "CBD" may also represent the abbreviation for cell wall binding domain. The terms "cell binding domain" and "cell wall binding domain" may be used interchangeably.

Specifically, a "bacteriophage lysin" according to the present invention is a combination of a first portion and a second portion, wherein the first portion comprises an amino acid sequence of a bacteriocin cell binding domain (CBD) and the second portion comprises at least one enzymatic active domain (EAD), wherein the domains stem from a different source organism, or source enzyme. In other words, the domains stem from a different origin of organism or different origin of enzyme. In this context, the terms "protein" and" peptide" may be used interchangeably with the term "enzyme". In other words, a "chimeric polypeptide" of the present invention is a polypeptide that comprises heterologous domains.

In the present invention, the bacteriophage lysin comprises a first and a second portion, wherein the first portion generally comprises an amino acid sequence of a bacteriocin CBD. Bacteriocins are molecules produced by microorganisms. Thus, if the second portion of the chimeric polypeptide comprises an amino acid sequence of the lytic domain of a bacteriophage lysin, such as the EAD, the chimeric polypeptide is chimeric because the CBD stems from a microorganism, while the EAD stems from a bacteriophage.

In one embodiment of the present invention, the bacteriophage lysin is a polypeptide comprising a first portion and a second portion joined by a linker, wherein said first portion comprises an amino acid sequence of a bacteriocin cell-binding domain (CBD) and said second portion comprises an amino acid sequence of an enzymatic active domain (EAD). Preferably, the EAD is the lytic domain of a bacteriophage endolysin.

More specifically, a bacteriophage lysin according to the invention is a combination of a first portion and a second portion, wherein the first portion comprises an amino acid sequence of a bacteriocin cell-binding domain (CBD) and the second portion comprises at least one enzymatic active domain (EAD), wherein the EAD is the lytic domain of a bacteriophage lysin. Preferably, the EAD is the lytic domain of a bacteriophage endolysin. More preferably, the EAD is the lytic domain of a bacteriophage endolysin, wherein the bacteriophage is from a Gram-positive bacterium. Still more preferably, the EAD is the lytic domain of a bacteriophage endolysin, wherein the bacteriophage is from a species or sub-species of *Staphylococcus.* Still more preferably, the EAD is the lytic domain of lysK, specifically the CHAP domain of lysK. Most preferably, the EAD comprises the amino acid sequence shown in SEQ ID NO: 1.

Thus, in a further embodiment, the present invention relates to a composition as disclosed herein, wherein the bacteriophage lysin is a polypeptide comprising an EAD that has at least 80%, preferably 90%, amino acid sequence identity with the polypeptide of SEQ ID NO: 1.

The chimeric polypeptide of the present invention comprises a first portion and a second portion joined by a linker, wherein said first portion comprises an amino acid sequence of a bacteriocin cell-binding domain (CBD). Preferably, the bacteriocin CBD of the present invention is a CBD produced by a Gram-positive bacterium. More preferably, the bacteriocin CBD of the present invention is a *Staphylococcus* bacteriocin CBD. Still more preferably, the bacteriocin CBD is the CBD of lysostaphin. The bacteriocin lysostaphin is naturally produced by *Staphylococcus simulans.* Most preferably, in the present invention the bacteriocin CBD comprises the amino acid sequence of SEQ ID NO: 2.

Thus, in a further embodiment, the present invention relates to a composition as disclosed herein above, wherein the bacteriophage lysin is a polypeptide comprising a CBD that has at least 80%, preferably 90%, amino acid sequence identity with the polypeptide of SEQ ID NO: 2.

One particularly useful bacteriophage lysin in the context of the present invention is the phage lysin, herein after referred to as HY-133. HY-133 is a recombinantly produced chimeric bacteriophage lysin composed of two polypeptide domains (SEQ ID NOs: 1 and 2) joined by a linker. The full-length amino acid sequence of HY-133 is shown in SEQ ID NO: 5. Domain 1 of HY-133 is the enzymatically active domain, which cleaves the cell wall of *S*. *aureus,* and domain 2 of HY-133 is the binding domain, which specifically binds to the cell wall of said bacterium. As used herein, HY-133 is provided as a non-exclusive example for the purpose of describing embodiments of the invention, and should not be construed as limiting the invention. Further Examples of bacteriophage lysins useful in the context of the present invention are disclosed in EP 2 338 916 and EP 2 516 471, the content of which is herein incorporated by reference.

In a further embodiment, the present invention relates to a composition as disclosed herein, wherein the bacteriophage lysin is a polypeptide that has at least 80%, preferably 90%, amino acid sequence identity with the polypeptide of SEQ ID NO: 5.

The term "linker", as used herein, refers to an amino acid sequence that joins the two portions of the chimeric polypeptide, or fragments or variants thereof. In general, as used herein, a linker is an amino acid sequence that covalently links the polypeptides, specifically the EAD and CBD, to form a fusion polypeptide. The linker comprises at least one peptide bond. As would be appreciated by one of skill in the art, the linker can comprise additional amino acids, such as glycine and other small neutral amino acids.

### Compositions/therapeutic formulations

For the purpose of the present invention, the active agent as described herein above is formulated for topical administration, preferably in liquid or semi-solid dosage form. Thus, a high level of stability of the active agent in solution has to be guaranteed during the time of storage and use.

The terms "topical" or "topical administration", as used herein, are meant to encompass local administration of a composition of the invention, in particular a fluid composition, to the surface of a skin or mucosal tissue of a subject without inducing any systemic effect, including parenteral, nasal and cutaneous administration. The terms "cutaneous" or "cutaneous administration" are, in the context of the present invention, meant to include administration to the surface of a skin or mucosal tissue, such as topical administration to the skin for the local treatment of a disease of the skin.

The compositions of the invention can be administered or applied to a subject by any suitable means. Means of application of the composition of the invention include, but are not limited to, liquid or semi-solid dosage forms; such as , e.g. solutions, gels, suspensions, gel-like solutions or gel-like suspensions, ointments, creams, emulsions, suspensions, foams, aerosols, nasal sprays or drops, or the like, or any other formulation known to a person skilled in the art. The composition of the invention may be formulated in a solid dosage form such as lyophilisates, which may be combined with an appropriate fluid before administration. It is most probable that exposure to the bacteria will be through the nose or skin. Preferred are sprays, liquids, gels, ointments, and aerosols. Particularly preferred are liquids, gels and ointments, including emulsions. Most preferred are liquids and gels.

Said dosage forms, as described herein in the context of the invention, serve as carriers for the therapeutic protein that is topically delivered. Examples for an appropriate route of administration are topical administration by way of the skin and mucosa, e.g., nasal mucosa, buccal tissue, cornea, rectal tissue, urethral membrane, vagina, external ear lining etc.

In a further embodiment of the invention, the composition of the invention is in form of a solution, a gel-like preparation, a semi-solid or solid preparation, a sprayable preparation or a lyophilizate.

The compositions of the invention are preferably pharmaceutical compositions. As used herein, a "pharmaceutical composition" refers to a composition that is pharmaceutically acceptable. As used herein, the term "pharmaceutically acceptable" refers to those compounds, materials, compositions, and/or dosage forms that are, within the scope of sound medical judgment, suitable for contact with the tissues of human beings and animals without excessive toxicity, irritation, allergic response, or other complications commensurate with a reasonable benefit/risk ratio.

The composition of the invention optionally comprises one or more pharmaceutically acceptable carriers or excipients.

As used herein, "carriers and excipients" refers to substances that are used in the formulation of the pharmaceutical compositions, and, by themselves, generally have little or no therapeutic value. Typical excipients include solvents, oils, antioxidants, salts, metal-ions, surfactants, anti-bacterial agents and other preservatives; chelating agents; buffering agents; agents for adjusting tonicity; colouring, flavouring and diluting agents; emulsifying and suspending agents; and other substances with pharmaceutical applications. Non-limiting examples for pharmaceutically acceptable "carriers and excipients" suitable for use in the compositions of the invention are antioxidants; gel-forming, specifically hydrogel-forming substances and buffering agents. Further examples are surfactants and salts used for tonicifying and enhancing colloidal stability, as well as special ligands, such as metal ions for use in the enzymatic active area.

As used herein, "antioxidants" in this context means substances that inhibit or delay the oxidation of biologically relevant molecules, e.g., by specifically quenching free radicals or by chelation of redox metals. Examples are methionine and cysteine.

As used herein, "hydrogel-forming substances" refers to substances formed when an organic polymer (natural or synthetic) is crosslinked via covalent, ionic, or hydrogen bonds to create a three-dimensional structure that entraps or bonds with water molecules or activating fluid, such as aqueous fluid. Examples for "hydrogel-forming substances" are hydroxyethyl cellulose, starch, carmellose and alginate, as well as Poloxamers.

"Poloxamers" as used herein are block copolymers of poly(ethylene oxide) and polypropylene oxide), well-known as non-ionic surfactants that, in high concentrations, form aqueous gels that undergo transitions from a low to a high viscous state as a consequence of an increase in temperature, known as "thermal gelation". Poloxamers are also commonly used as surfactants in protein formulation, and thereby reduce aggregation and air-water interface interaction. A preferred example is Poloxamer 188.

In one embodiment of the invention, the composition of the invention comprises one or more piperazine- or morpholine-containing zwitterionic buffering substance, preferably 2-(4-(2-hydroxyethyl)-1-piperazinyl)-ethane sulfonic acid (HEPES) and one or more pharmaceutically acceptable carriers or excipients as defined herein. Examples for pharmaceutically acceptable carriers or excipients are CaCl₂, NaCl, and Arginine-HCl.

In a further embodiment of the invention, the composition of the invention comprises 2-(4-(2-hydroxyethyl)-1-piperazinyl)-ethane sulfonic acid (HEPES), but does not comprise additional buffers.

The pH value of the composition of the invention can be in the range of about pH 4 to about 8, preferably in the range of about 4.5 to about 7.5, more preferably in the range of about 5 to about 7, still more preferably in the range of about 5.5 to about 6.5, and most preferably about 6. As used herein, the term "about" refers to a range of values + 5 % of a specified value. For example, the phrase "about 6" includes ± 5% of 6, or from 5.7 to 6.3.

In a further embodiment of the invention, the composition of the invention is in a liquid or semi-solid dosage form, preferably a solution comprising HEPES, wherein the pH value of the solution is in the range of pH 4-8.

In a preferred embodiment of the invention, the composition of the invention is in a liquid or semi-solid dosage form, preferably a solution comprising HEPES, wherein the pH value of the solution is in the range of pH 5-7.

In a more preferred embodiment of the invention, the composition of the invention is in a liquid or semi-solid dosage form, preferably a solution comprising HEPES, wherein the pH value of the solution is in the range of pH 5.5-6.5.

In a still more preferred embodiment, the composition of the invention is in a liquid or semi-solid dosage form, preferably a solution comprising HEPES, wherein the pH value of the solution is in the range of pH 4-8, preferably in the range of pH 5-7, more preferably in the range of 5.5-6.5, and the composition comprises one or more other carriers or excipients as defined herein.

In a still more preferred embodiment, the composition of the invention as described herein above comprises the bacteriophage lysin and one or more antioxidants, preferably Methionin, and one or more other excipients, preferably selected from the group consisting of CaCl₂, NaCl, Arginin-HCl, and Poloxamer 188.

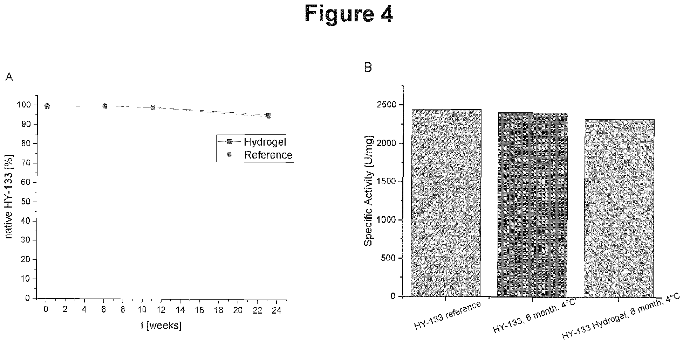

In a still more preferred embodiment, the composition of the invention as described herein above comprises the bacteriophage lysin, HEPES, Methionin, CaCl₂, NaCl, and Arginin-HCl, and the pH of the solution is about pH 6. For example, an optimal combination is as follows: 0.5mg/ml HY-133 in 25 mM HEPES, 75-150 mM NaCl, 150-300 mM Arg-HCl, 10 mM CaCl₂ and 10 mM Methionin at pH 6.0. In this composition, more than 95% of native protein was available after storage for six months at 4°C (see Figure 4).

The concentration of piperazine- or morpholine-containing zwitterionic buffering substances according to the invention is in the range of about 0.01 to about 1000 mM, preferably in the range of about 1 to about 400 mM, more preferably in the range of about 10 to about 100 mM, still more preferably in the range of about 10 to about 50 mM, still more preferably in the range of about 20 to about 50 mM, still more preferably in the range of about 10 to about 40 mM, even more preferably in the range of about 20 to about 40 mM. As used herein, the term "about" refers to a range of values + 5% of a specified value. For example, the phrase "about 20 to about 40" includes ± 5% variation, i.e. 19 to 42.

In one embodiment of the invention, the piperazine- or morpholine-containing zwitterionic buffering substance is HEPES, and the concentration is in the range of 0.01-1000 mM.

In a preferred embodiment of the invention, the piperazine- or morpholine-containing zwitterionic buffering substance is HEPES, and the concentration is in the range of 1-400 mM.

In a more preferred embodiment of the invention, the piperazine- or morpholine-containing zwitterionic buffering substance is HEPES, and the concentration is in the range of 10-100 mM.

In a still more preferred embodiment of the invention, the piperazine- or morpholine-containing zwitterionic buffering substance is HEPES, and the concentration is in the range of 20-40 mM.

In a particular preferred embodiment of the composition of the invention, the composition is in the form of a fluid that comprises one or more thickening agents.

As used herein, the term "thickening agent" refers to a substance which, when added to various blends of aqueous and non-aqueous solutions comprising the composition of the invention, increases the viscosity of said solution without substantially affecting the chemical and physical stability of the composition or the biological activity of the active ingredients present therein.

The term "gel-forming substance", as used herein, refers to polymers that are capable of forming three-dimensional networks upon cross-linking and the subsequent absorption of water. Examples of thickening and swelling agents include, but are not limited to, hydroxypropyl methylcellulose (short form: HPMC or hypromellose), methyl cellulose, hydroxypropyl cellulose, hydroxyethyl cellulose, carboxymethyl cellulose, alginates, xanthan gums, Xellan gums, Gummi arabicum, pectins, and other polysaccharide-based gel-formers or starches.

In a preferred embodiment of the invention, HPMC is included in the composition. HPMC is a semisynthetic, inert, viscoelastic polymer commonly used as an excipient in ophthalmic preparations or pharmaceutical compositions for oral administration. HPMC is available in different viscosity grades; from low viscosity grade HPMC K4M to higher viscosity grade HPMC K100M.

For the purpose of this invention, the HPMCs are preferably those that are of a low-viscosity grade or are of low viscosity. "Low-viscosity grade" implies those cellulose ethers, which, when in a 2 weight percent aqueous solution, exhibit a viscosity at 20°C below about 10,000 cP (10,000 mPa-s). Conversely, "high-viscosity grade" or "high viscosity" implies those cellulose ethers which, when in a 2 weight percent aqueous solution, exhibit a viscosity at 20°C of greater than about 10,000 centipoise (cP) (10,000 mPa-s), and may have a viscosity as high as 2,000,000 cP (2,000,000 mPa-s).

The term "viscoelastic polymer solution", as used herein, refers to a liquid of an ultrahigh molecular weight polymer exhibiting both viscous and elastic properties. A viscoelastic liquid will readily deform and flow under the influence of an applied shear stress. When the stress is removed, the liquid will quickly recover from the last small portion of the deformation. For the purpose of this invention, a composition comprising a polymer solution having 0.1-4% concentration by weight of ultrahigh molecular weight polymer will be considered a "gel-like composition." Example 5 demonstrates actual tests with a composition comprising a polymer solution as defined above, and the results of those tests. Thus, in a further embodiment of invention, the composition described herein is a gel-like preparation that comprises HPMC, preferably HPMC K4M, HPMC K15M or HPMC K100M, more preferably HPMC K4M. The designations HPMC K4M, HPMC K15M and HPMC K100M specify hydroxypropyl methylcellulose having the particular viscosity grade as indicated. Typical visocities as stated in the Certificate of Analysis by Dow Chemical are viscosity ranges of 2663-4970 mPa*s for HPMC K4M (Methocel K4M), 13275-24780 mPa*s for K15M and 75000-140000 mPa*s K100M, all referring to a 2% solution in water.

In a further embodiment of invention, the composition described herein is a gel-like preparation that comprises 0.1-4% HPMC, preferably 0.2-3.5% HPMC, more preferably 0.3-3.2% HPMC, still more preferably 0.4-3.0% HPMC, even more preferably 0.45-2.5% HPMC, most preferably 0.5-2% HPMC. In one embodiment of the invention, the HPMC included in the composition described above is HPMC K4M.

In a preferred embodiment of the invention, the composition described herein is a gel-like preparation that comprises 0.1-4% HPMC K4M. In a still more preferred embodiment of the invention, the composition described herein is a gel-like preparation that comprises 0.5-2% HPMC K4M.

The compositions and formulations comprising a chimeric polypeptide of the present invention as an active ingredient are applied in an effective amount when used in prophylaxis and therapy. The term "effective amount" refers to an amount of an active ingredient sufficient to achieve a desired effect without causing an undesirable side effect. In some cases, it may be necessary to achieve a balance between obtaining a desired effect and limiting the severity of an undesired effect. The amount of active ingredient used will vary depending upon the type of active ingredient and the intended use of the composition and/or formulation of the present invention.

In a further embodiment of invention, the composition described herein comprises a bacteriophage lysin concentration in the range of 0.01 mg/ml to 100 mg/ml, preferably in the range of 0.1 mg/ml to 20 mg/ml, more preferably in the range of 0.2 mg/ml to 10 mg/ml, even more preferably in the range of 0.4 mg/ml to 6 mg/ml, and most preferred in the range of 0.5 mg/ml to 2 mg/ml.

The compositions and formulations according to the present invention are particularly useful for the prophylaxis and treatment of upper respiratory infections, skin infections, wounds, burns, vaginal infections, eye infections, intestinal disorders and dental disorders. Specifically, the invention provides the application of the bacteriophage lysin for nasal and/or skin decolonisation of human and animals.

In a preferred embodiment of invention, the composition is provided for use in treating a bacterial infection or preventing or eliminating nasal bacterial colonization or bacterial colonization of the skin. In another embodiment, the compositions of the present invention are used for treating or ameliorating wounds. In a further embodiment of invention, the composition described herein is prepared for topical, cutaneous or nasal administration.

The present invention provides a method for treating a bacterial infection or preventing or eliminating nasal bacterial colonization or bacterial colonization of the skin, comprising administering an effective amount of the composition of the invention. The present invention further provides a method for treating or ameliorating wounds, comprising administering an effective amount of the composition of the invention.

In preferred embodiments, the compositions of the invention are used as a prophylactic treatment for preventing illness in subjects, preferably human subjects, who have possibly been exposed to *Staphylococcus* bacteria, or as a therapeutic treatment for those subjects, preferably human subjects, who have already become ill from an infection with *Staphylococcus* bacteria. The bacteriophage lysins included in the compositions of the invention are preferably specific for the decolonisation of *Staphylococcus* bacteria, and preferably effectively and efficiently break down the cell wall of *Staphylococcus* bacteria, preferably of *S*. *aureus,* more preferably of methicillin-resistant *S*. *aureus* (MRSA).

In a particular embodiment, the compositions of the present invention are used for medical treatment if the bacterial infection to be treated (or prevented) is caused by multiresistant *Staphylococcus* strains, in particular by strains resistant against vancomycin, linezolid or daptomycin.

The effective dosage rates or amounts of the bacteriophage lysin used to treat the infection will depend on whether the bacteriophage lysin is to be used therapeutically or prophylactically, the duration of exposure of the recipient to the infectious bacteria, the size and weight of the individual, etc. The duration for use of the composition containing the bacteriophage lysin also depends on whether the use is for prophylactic purposes (wherein the use may be hourly, daily or weekly, for a short time period), or whether the use will be for therapeutic purposes (wherein a more intensive regimen of the use of the composition may be needed, such that usage may last for hours, days or weeks, and/or on a daily basis, or at timed intervals during the day). Any dosage form employed should provide for a minimum number of units for a minimum amount of time.

The concentration of the active units of bacteriophage lysin that may provide for an effective amount or dosage of the bacteriophage lysin may be in the range of 10 units/ml to 500,000 units/ml of a formulation for nasal or topical administration. Representative values thus include about 200 units/ml, 300 units/ml, 500 units/ml, 1,000 units/ml, 2,500 units/ml, 5,000 units/ml, 10,000 units/ml, 20,000 units/ml, 30,000 units/ml, and 40,000 units/ml. More specifically, time exposure to the active enzyme units may influence the desired concentration of active enzyme units per ml. The number of dosages will be dependent upon the circumstances and can range from one to four times per day or more, with durations from one day to multiple weeks.

In a further embodiment, the present invention provides a method for preparing a composition, preferably a pharmaceutical composition, for topical administration of a bacteriophage lysin as defined herein above, which comprises the step of mixing the bacteriophage lysin solution with a zwitterionic buffering substance and optionally adding a pharmaceutically acceptable carrier or excipient.

The present invention is more particularly described in the following, non-limiting examples, which are intended to be illustrative only, as numerous modifications and variations therein will be apparent to those skilled in the art.

### EXAMPLES

### Example 1: Materials and Methods

### Preparation of dilution buffers:

A mixture of 25 mM HEPES, 75 or 150 mM NaCl, 150 or 300 mM Arg-HCl, 10 mM CaCl₂ and 10 mM Methionin were diluted in highly purified water. The pH was adjusted to 6.0 followed by filtration with a 0.22 µm filter (e.g. PES or PVDF).

### Preparation of solutions:

Five different formulations were prepared as follows: Freshly thawed HY-133 was diluted with dilution buffer to obtain a protein solution of 0.5 mg/ml. As a control, solutions of freshly thawed HY-133 were prepared.

As the pH of about 6.0 for the HY-133 formulation is not in the optimal pH range of HEPES, control formulations containing citrate and histidine buffers, which are typically used in this pH range, are tested.

The formulations containing citrate and histidine buffers were prepared by removing HEPES by means of dialysis, and replacing HEPES with either 25 mM citrate buffer or 25 mM histidine buffer. Additionally, combinations of HEPES buffer and either citrate or histidine buffer were prepared.

### Stability studies:

Stability of HY-133 formulations at various storage temperatures (4°C, 40°C) over a time of up to 16 months was observed with various analysis techniques.

### RP-HPLC:

For evaluating the chemical stability of HY-133, HY-133 is separated in an acetonitrile gradient (10-100%, 22 min) by using RP-HPLC, specifically a Phenomenex Jupiter C4 300 Å 250 x 4.6 mm column, and detected via fluorescence detection at 280/343 nm. Chemically modified HY-133 eluted earlier than native HY-133.

### SE-HPLC:

For evaluating the degree of fragmentation, the proteins were separated using SEC, specifically a TOSOH TSKgel G3000SWXL 300 x 7.8 mm in a buffer comprising 50 mM Na₃PO₄ and 300 NaCl at pH 7 for a period of 45 min, and detected via UV absorption at 280nm.

### Activity:

The activity was determined by using a specific activity assay provided by Microcoat (Bernried, Germany). A FRET molecule that mimics the peptidoglycan structure was cleaved by HY-133 and the resulting fluorescence was measured.

### Example 2:

The chemical degradation of HY-133 in various buffer systems was detected by RP-HPLC. A very good stability at 4°C was observed in all compositions comprising HEPES. A pure histidine- or citrate buffer leads to degradation of the native proteins. An accelerated stability study at 40°C shows that HEPES buffer formulations significantly reduce chemical degradation compared to histidine or citrate formulations. Buffer combinations composed of HEPES and histidine or HEPES and citrate show improved stability compared to the individual histidine and citrate buffers, but do not achieve the greatly improved stability of pure HEPES formulations.

The use of histidine or citrate as buffer in the formulation results in significant chemical degradation of native HY-133 during both moderate storage at 4°C and stability-enhancing storage at 40°C. The addition of HEPES to histidine or citrate containing formulations enhances chemical stability of HY-133 and results at 4°C in formulations with similar stability as the HEPES buffer alone.

The results surprisingly demonstrated that HEPES is needed for the chemical stability of HY-133, specifically for the avoidance of oxidation, although the problem of oxidation has already been addressed by 10 mM Methionin (Hada *et al.,* 2016). For protein formulations, a concentration of 10 mM Methionin is usually considered sufficient to prevent chemical degradation, but was insufficient to stabilize HY-133. Only the combination of both excipients leads to a stabilized formulation. The addition of HEPES could largely prevent degradation of HY-133.

### Example 3:

The degree of fragmentation was determined after storage for five weeks at 40°C. For any formulation except the citrate buffer-containing formulation, a degree of fragmentation was observed, which was in an acceptable range. However, the citrate buffer-containing formulation caused a strong increase in fragments. This high degree of fragmentation can be avoided by using a combination of citrate and HEPES.

Moreover, the use of HEPES in formulations containing HY-133 surprisingly resulted in a more significant improvement of physical stability, even in the presence of various salts (CaCl₂, Arg-HCl, NaCl), which also have a significant influence on physical stability. The use of histidine as a buffer resulted in a high degree of fragmentation after storage for five weeks at 40°C. This could not be observed with formulations containing citrate, HEPES, or a combination thereof. It is noteworthy that the addition of HEPES to the histidine buffer largely prevents the formation of fragments. Even if one considers the adverse effects of histidine, significantly better results could be achieved due to the positive effect of HEPES.

### Example 4:

The activity of the various formulations was determined after storage for five weeks at 40°C, thereby a different change of activity of HY-133 was observed in the different buffer systems. The activity decreases more significantly in pure histidine- or citrate buffers than in HEPES-containing formulations. The determination of the activity can be conducted during the preparation process, since activity in all HEPES-containing formulations is much higher compared to formulations with pure histidine- or citrate buffers.

The results show that the selection of buffer substances has a significant effect on the specific activity of HY-133. Initially, both the citrate buffer and the histidine buffer show a slightly lower activity as compared to the HEPES- or combined HEPES formulations. This effect was significantly higher after storage at 40°C for five weeks. The activity of HY-133 in citrate or histidine formulations decreases rapidly, whereas HEPES-containing formulation showed only a slight decrease. Therefore, it must be concluded that HEPES is responsible for the stability of HY-133.

### Example 5:

The activity of a gel-like preparation using HPMC was determined after storage for six months at 4°C, which shows that no additional chemical degradation occurs compared to the liquid reference sample of the above-mentioned ideally suitable formulation containing HEPES. In addition, no negative effect on the activity of HY-133 after storage for six months at 4°C owing to the addition of a gel-forming substance was observed for HPMC. Formulations using NaCMC as a gelling agent lead to accelerated degradation in short term stability studies.

### Example 6:

For nasal application, the above-mentioned gel-like preparation of HY-133 may be dispensed and sprayed using customary primary packaging materials, which allow for nasal application of the active agent. For topical application, the formulation may also be in the form of a sprayable formulation.

### References

Banga, A. (2006). Therapeutic Peptides and Proteins: Formulation, Processing, and Delivery Systems / A.K. Banga. https://doi.org/10.1201/9781420039832
Hada, S., Kim, N. A., Lim, D. G., Lim, J. Y., Kim, K. H., Adhikary, P., & Jeong, S. H. (2016). Evaluation of antioxidants in protein formulation against oxidative stress using various biophysical methods. International Journal of Biological Macromolecules, 82, 192-200. https://doi.org/10.1016/j.ijbiomac.2015.10.048
Jiskoot, W., Randolph, T. W., Volkin, D. B., Middaugh, C. R., Schöneich, C., Winter, G., ... Carpenter, J. F. (2012). Protein instability and immunogenicity: Roadblocks to clinical application of injectable protein delivery systems for sustained release. Journal of Pharmaceutical Sciences, 101(3), 946-954. https://doi.org/10.1002/jps.23018
Jorgensen, L., Hostrup, S., Moeller, E. H., & Grohganz, H. (2009). Recent trends in stabilising peptides and proteins in pharmaceutical formulation - considerations in the choice of excipients. Expert Opinion on Drug Delivery, 6(11), 1219-1230. https://doi.org/10.1517/17425240903199143
Kamerzell, T. J., Esfandiary, R., Joshi, S. B., Middaugh, C. R., & Volkin, D. B. (2011). Protein-excipient interactions: mechanisms and biophysical characterization applied to protein formulation development. Advanced Drug Delivery Reviews, 63(13), 1118-1159. https://doi.org/10.1016/j.addr.2011.07.006
Shire, S. J. (2015). Monoclonal Antibodies. Monoclonal Antibodies. Elsevier. https://doi.org/10.1016/B978-0-08-100296-4.00002-6
Zbacnik, T. J., Holcomb, R. E., Katayama, D. S., Murphy, B. M., Payne, R. W., Coccaro, R. C., ... Manning, M. C. (2017). Role of Buffers in Protein Formulations. Journal of Pharmaceutical Sciences, 106(3), 713-733. https://doi.org/10.1016/j.xphs.2016.11.014

Further aspects and embodiments of the invention include:
1. A composition for topical administration of a bacteriophage lysin, preferably a pharmaceutical composition, comprising:
   (I) a bacteriophage lysin as an active ingredient,
   (II) a piperazine- or morpholine-containing zwitterionic buffering substance, and
   (III) optionally a pharmaceutically acceptable carrier or excipient.
2. The composition of item 1, wherein the piperazine- or morpholine-containing zwitterionic buffering substance is selected from the group consisting of 2-(4-(2-hydroxyethyl)-1-piperazinyl)-ethane sulfonic acid (HEPES), piperazine-1,4-bis(2-hydroxy-3-propane sulfonic acid) dehydrate (POPSO), 2-(N-morpholino)-ethane sulfonic acid (MES) and 3-(N-morpholino)-propane sulfonic acid (MOPS), preferably wherein the composition comprises HEPES as the sole buffer.
3. The composition of item 1 or 2, wherein the bacteriophage lysin is a polypeptide comprising a first portion and a second portion joined by a linker, wherein said first portion comprises an amino acid sequence of a bacteriocin cell-binding domain (CBD), preferably a lysostaphin CBD and said second portion comprises an amino acid sequence of an enzymatic active domain (EAD), preferably the lytic domain of a bacteriophage endolysin, more preferably lysK.
4. The composition of item 3 , wherein the lytic domain has at least 80%, preferably 90%, amino acid sequence identity with the polypeptide of SEQ ID NO: 1, and/or, wherein the CBD has at least 80%, preferably 90%, amino acid sequence identity with the polypeptide of SEQ ID NO: 2.
5. The composition of any one of items 1 to 4, wherein the bacteriophage lysin is a polypeptide having at least 80%, preferably 90%, amino acid sequence identity with the polypeptide of SEQ ID NO: 5.
6. The composition of any one of items 1 to 5, wherein the composition is in form of a solution, a gel-like preparation, a solid preparation, a sprayable preparation or a lyophilisate.
7. The composition of claims of any one of items 1 to 6, wherein the composition is in form of a solution comprising HEPES and one or more other excipients, and wherein the pH value of the solution is in the range of pH 4-8, preferably in the range of pH 5-7, more preferably in the range of pH 5.5-6.5.
8. The composition of any of items 1 to 7, wherein the composition further comprises at least one antioxidant, preferably Methionin, and one or more other excipients, preferably selected from the group consisting of CaCl₂, NaCl, Arginin-HCl, and optionally Poloxamer 188, preferably wherein the composition comprises HEPES, Methionin, CaCl₂, NaCl, and Arginin-HCl, and the pH of the solution is pH=6.
9. The composition of any one of items 2 to 8, wherein the HEPES concentration is in the range of 0.01-1000 mM, preferably in the range of 1-400 mM, more preferably in the range of 10-100 mM, even more preferably in the range of 20-40 mM.
10. The composition of any one of items 1 to 9, wherein the composition further comprises HPMC, preferably HPMC K4M, K15M or K100M, more preferably wherein the composition comprises 0.1-4% per weight HPMC, preferably 0.5-2 % per weight HPMC K4M.
11. The composition of any one of items 1 to 10, wherein the bacteriophage lysin concentration is in the range of 0.01 mg/ml to 100 mg/ml, preferably in the range of 0.1 mg/ml to 20 mg/ml, more preferably in the range of 0.2 mg/ml to 10 mg/ml, even more preferably in the range of 0.4 mg/ml to 6 mg/ml, and most preferred in the range of 0.5 mg/ml to 2 mg/ml.
12. The composition of any one of items 1 to 11, wherein the composition is prepared for cutaneous or nasal administration.
13. The composition according to any one of items 1 to 12 for use in treating a bacterial infection or preventing or eliminating nasal bacterial colonization or skin colonization.
14. The composition according to any one of items 1 to 13 for use in treating MRSA or for ameliorating wounds.
15. A method for preparing a composition for topical administration of a bacteriophage lysin according to any of items 1 to 11 comprising the step of mixing a bacteriophage lysin solution with a zwitterionic buffering substance and optionally adding a pharmaceutically acceptable carrier or excipient.

## Claims

1. A composition for topical administration of a bacteriophage lysin, preferably a pharmaceutical composition, comprising:
(I) a bacteriophage lysin as an active ingredient,
(II) a piperazine- or morpholine-containing zwitterionic buffering substance, and
(III) optionally a pharmaceutically acceptable carrier or excipient.

2. The composition of claim 1, wherein the piperazine- or morpholine-containing zwitterionic buffering substance is selected from the group consisting of 2-(4-(2-hydroxyethyl)-1-piperazinyl)-ethane sulfonic acid (HEPES), piperazine-1,4-bis(2-hydroxy-3-propane sulfonic acid) dehydrate (POPSO), 2-(N-morpholino)-ethane sulfonic acid (MES) and 3-(N-morpholino)-propane sulfonic acid (MOPS), preferably wherein the composition comprises HEPES as the sole buffer.

3. The composition of claim 1 or 2, wherein the bacteriophage lysin is a polypeptide comprising a first portion and a second portion joined by a linker, wherein said first portion comprises an amino acid sequence of a bacteriocin cell-binding domain (CBD), preferably a lysostaphin CBD and said second portion comprises an amino acid sequence of an enzymatic active domain (EAD), preferably the lytic domain of a bacteriophage endolysin, more preferably lysK.

4. The composition of claim 3 , wherein the lytic domain has at least 80%, preferably 90%, amino acid sequence identity with the polypeptide of SEQ ID NO: 1, and/or, wherein the CBD has at least 80%, preferably 90%, amino acid sequence identity with the polypeptide of SEQ ID NO: 2.

5. The composition of any one of claims 1 to 4, wherein the bacteriophage lysin is a polypeptide having at least 80%, preferably 90%, amino acid sequence identity with the polypeptide of SEQ ID NO: 5.

6. The composition of any one of claims 1 to 5, wherein the composition is in form of a solution, a gel-like preparation, a solid preparation, a sprayable preparation or a lyophilisate.

7. The composition of any one of claims 1 to 6, wherein the composition is in form of a solution comprising HEPES and one or more other excipients, and wherein the pH value of the solution is in the range of pH 4-8, preferably in the range of pH 5-7, more preferably in the range of pH 5.5-6.5.

8. The composition of any one of claims 1 to 7, wherein the composition further comprises at least one antioxidant, preferably Methionin, and one or more other excipients, preferably selected from the group consisting of CaCl₂, NaCl, Arginin-HCl, and optionally Poloxamer 188, preferably wherein the composition comprises HEPES, Methionin, CaCl₂, NaCl, and Arginin-HCl, and the pH of the solution is pH=6.

9. The composition of any one of claims 2 to 8, wherein the HEPES concentration is in the range of 0.01-1000 mM, preferably in the range of 1-400 mM, more preferably in the range of 10-100 mM, even more preferably in the range of 20-40 mM.

10. The composition of any one of claims 1 to 9, wherein the composition further comprises HPMC, preferably HPMC K4M, K15M or K100M, more preferably wherein the composition comprises 0.1-4% per weight HPMC, preferably 0.5-2 % per weight HPMC K4M.

11. The composition of any one of claims 1 to 10, wherein the bacteriophage lysin concentration is in the range of 0.01 mg/ml to 100 mg/ml, preferably in the range of 0.1 mg/ml to 20 mg/ml, more preferably in the range of 0.2 mg/ml to 10 mg/ml, even more preferably in the range of 0.4 mg/ml to 6 mg/ml, and most preferred in the range of 0.5 mg/ml to 2 mg/ml.

12. The composition of any one of claims 1 to 11, wherein the composition is prepared for cutaneous or nasal administration.

13. The composition according to any one of claims 1 to 12 for use in treating a bacterial infection or preventing or eliminating nasal bacterial colonization or skin colonization.

14. The composition according to any one of claims 1 to 13 for use in treating MRSA or for ameliorating wounds.

15. A method for preparing a composition for topical administration of a bacteriophage lysin according to any of claims 1 to 11 comprising the step of mixing a bacteriophage lysin solution with a zwitterionic buffering substance and optionally adding a pharmaceutically acceptable carrier or excipient.
